# EUROPEAN PATENT SPECIFICATION

(11) **EP 1 727 585 B1**
(45) Date of publication and mention of the grant of the patent: **13.10.2010**
(21) Application number: 05729196.5
(22) Date of filing: 16.03.2005
(51) Int. Cl.: A61M 25/01

(54) **SECOND WIRE APPARATUS AND INSTALLATION PROCEDURE**
ZWEITDRAHT-GERÄT UND INSTALLATIONSVERFAHREN
APPAREIL FIL-GUIDE SECONDAIRE ET PROCEDURE D'INSTALLATION APPROPRIEE

(30) Priority: 17.03.2004 US 554048 P; 07.12.2004 US 633749 P
(43) Date of publication of application: 06.12.2006
(73) Proprietor: Cook Incorporated, Bloomington, Indiana 47404 (US)
(72) Inventor: LORENZ, Mark, A., Peru, IN 46970 (US)
(74) Representative: Garratt, Peter Douglas
(86) International application number: PCT/US2005/008674
(87) International publication number: WO 2005/089852

(56) References cited:
- EP-A- 1 201 260
- EP-A- 1 240 915
- WO-A-93/13827
- US-A1- 2003 028 127
- PATENT ABSTRACTS OF JAPAN vol. 2003, no. 04, 2 April 2003 (2003-04-02) & JP 2002 355310 A (KAWASUMI LAB INC), 10 December 2002 (2002-12-10)

## Description

### TECHNICAL FIELD

This invention relates generally to a second wire apparatus and its installation in the PTCA (percutaneous transluminal coronary angioplasty) process. More specifically, this invention relates to the construction of the distal end of the second wire apparatus and a system for guiding it down an earlier installed guide wire to assist in balloon angioplasty in vessels with proximal tortuosity or as a more substantial guide wire for atherectomy devices, stents, stent delivery devices, lasers or other medical catheter devices.

### BACKGROUND OF THE INVENTION

In the past, balloon angioplasty in vessels with proximal tortuosity was associated with a higher incidence of acute complications and procedural failure due to the inability to cross lesions with a guide wire and inadequate guiding catheter support. Low profile balloons, extra support hydrophilic guide wires, and geometric guiding catheters have improved the results of PTCA in these and other challenging lesions. Severe proximal tortuosity is problematic for all atherectomy devices, stents, stent delivery devices, lasers, and other medical catheter devices which are bulky, less flexible, and less trackable than typical balloon catheters.

Most guide wires have a lubricous coating to enhance guide wire movement and are typically 0,36 mm (0.014") in diameter. Conventional 0,36 mm (0.014") floppy guide wires may be sufficient for tortuous vessels, but in some situations where the guide wire tip may prolapse away from the target lesion, a tapered core guide wire may be better utilized. In some cases, steerability and tip-response are lost as the guide wire passes through multiple curves or restrictions within the vessel. One option that has been used with limited success is to install a stiffer tip guide wire to improve handling characteristics. Another option, which is in the arena of this invention, is to add extra support guide wires in which the shaft, rather than the tip, is stiffer to straighten the vessel curves and ease guide wire movement. Although heavy-duty guide wires are generally not well suited as primary guide wires because of their stiffness and poor torque control, they provide excellent support and will enhance the tracking of balloons, stents, stent delivery devices, atherectomy devices, and other medical catheter devices when other guide wires fail. However, the feeding of this second stiffer guide wire parallel to the first guide wire is an exacting and time consuming process in which the second guide wire can corkscrew or coil around the first guide wire, which may result in unintended movement of the first guide wire or require the retraction and re- feeding of the second guide wire. Moreover, if retraction of the second guide is necessary, the guide wire may become contaminated and the entire process may need to be restarted with sterile components. The time consumed by this process can be critical to the success of the procedure.

Reference is directed to US 2003/028127 which discloses devices for coupling guide wires in guidance extension systems. In one disclosed arrangement, the shaped end of one guide wire is received with a T-shaped opening formed at the end of a second guide wire. This document discloses the features of the preamble of claim 1.

### BRIEF SUMMARY

An object of the present invention is to provide a second guide wire apparatus and installation procedure which allows for use of stiffer wire, reduces or eliminates twisting and coiling about the first guide wire, and which permits the second guide wire to be fed rapidly down (along) the first (in-place) guide wire. A second objective is to provide a second guide wire apparatus which can be fed all the way to the distal end of the first guide wire and then released from the first guide wire so that the first guide wire can be removed or advanced to the next stenosis if so desired.

A third objective is to provide a second guide wire apparatus that is sufficiently longer than the first (in-place) guide wire so that the distal ends of the guide wires may be disengaged from each other at the point where the proximal ends of the guide wires are equidistant from entering the body or are disposed adjacent to each other. This arrangement eliminates the need for radiopaque markers disposed on the guide wires at or near the distal ends thereof for monitoring their respective locations, with expensive and time consuming x-ray fluorescence, to determine when the first and second guide wires have become disengaged from each other.

### BRIEF DESCRIPTION OF THE DRAWINGS

In order that the invention may be more fully understood, it will now be described, by way of example, with reference to the accompanying drawings in which:

Figure 1 is a top view of a second guide wire apparatus engaged with a first guide wire;

Figure 2 is a cross-sectional view of a second guide wire apparatus engaged with a first guide wire;

Figure 3 is a top view of an end effecter slide;

Figure 4 is a cross-sectional view the end effecter slide;

Figure 5 is a top view of a second guide wire apparatus in an initial or starting position ready to engage the proximal end of an installed first guide wire; and

Figure 6 is a cross sectional view of a second guide wire apparatus which has been moved distally past the distal end of an installed first guide wire and allowed to disengage and separate from the first guide wire.

### DETAILED DESCRIPTION OF THE DRAWINGS AND THE PRESENTLY PREFERRED EMBODIMENTS

In order that the invention may be more fully understood, it will now be described, by way of example, with reference to the following description in conjunction with the accompanying drawings.

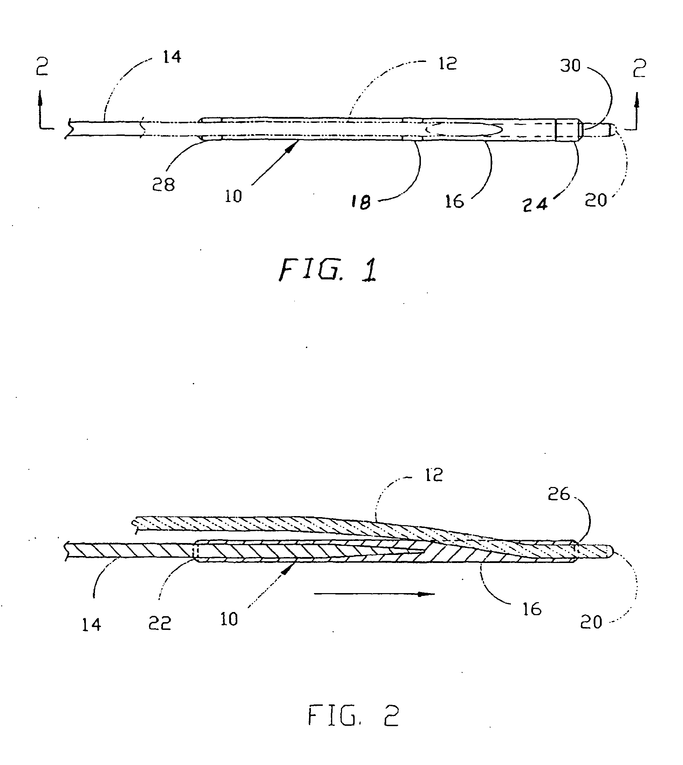

Figure 1 illustrates an embodiment of a second guide wire apparatus 10 according to the present invention which is engaged with an installed first guide wire 12. The installed first guide wire 12 has been inserted into a vessel of a patient (not shown) in a standard manner and advanced until the distal tip 20 of the installed first guide wire 12 is past the lesion or stenosis (not shown) being treated. Second guide wire apparatus 10 is shown as engaged with first guide wire 12 and approaching the distal tip 20 thereof.

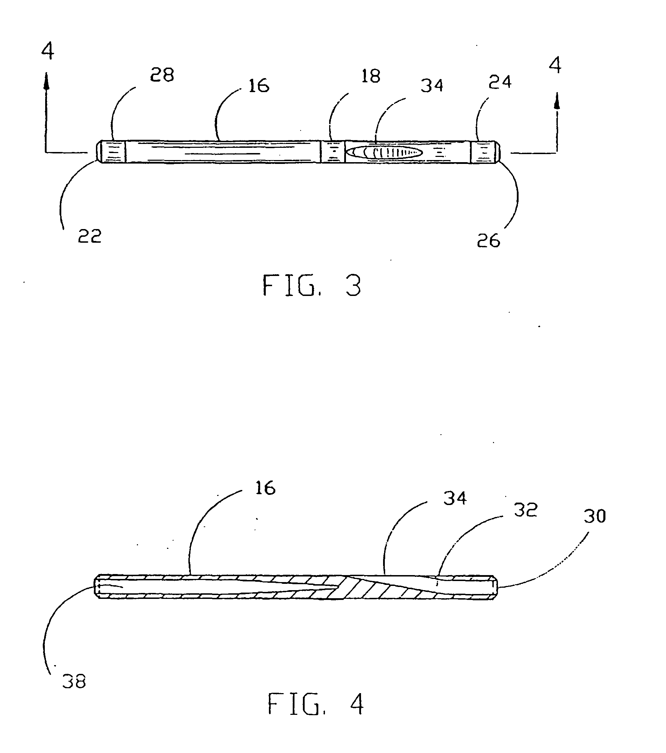

Figure 2 is a cross-sectional view of Figure 1 and illustrates the assembly of end effecter slide 16, also referred to as a coupling element, to the distal end portion of second support wire 14. In one embodiment, the end effecter slide 16 is fixedly attached or connected to the distal end of the second support wire 14 so as to form an integral structure. As will be explained in greater detail below, end effecter slide 16 comprises a guide wire channel or passageway 32 (see Figure 4) which allows the end effecter slide 16 to engage and be pushed along the installed first guide wire 12 until the end effecter slide 16 passes the lesion or stenosis (not shown).

Figure 3 is a top view of an embodiment of the end effecter slide 16 according to the present invention. The end effecter slide 16, also referred to as a coupling element, comprises a proximal (or intermediate) opening 34 that provides access to the proximal end of the guide wire channel 32 (Figure 4). The end effecter slide 16 is approximately 25 mm (1 inch) in length and 0.9 mm (0.035 inches) in diameter. As best seen in Figure 2, proximal opening 34 is configured to permit the installed first guide wire 12 to exit the guide wire channel 32 at a relatively close angle to the central axis of the end effecter slide 16, which minimizes the overall profile of the combined the second guide wire apparatus 10 and installed first guide wire 12. Proximal opening 34 is approximately 10 mm from the distal end of the end effecter slide 16. However, it should be understood that different dimensions can be utilized for the size and arrangement of any of these components depending on the size of the second support wire 14, the size of the installed first guide wire 12, and the medical procedure in which these guide wires are utilized. In the preferred embodiment illustrated, the end effecter slide 16 is made from a polytetrafluoroethylene (PTFE) material and has a low friction hydrophilic coating on the distal portion thereof, although it could be made from other materials such as any number of high temperature thermoplastics with a lubricious external surface. Figure 3 also illustrates chamfers 26 and 22 on the proximal and distale ends, respectively, of the end effecter slide 16. These chamfers 22 and 26 make passage of the end effecter slide 16 through a lesion or stenosis, in either direction, or around a tortuous bend in vessel less likely to cause or result in damage to the vascular wall.

In the embodiment illustrated, the end effecter slide 16 further comprises a radiopaque marker 24 at or near the distal end of the end effecter slide 16, a second radiopaque marker 18 at or near the rear edge of the proximal opening 34, and a third radiopaque marker 28 at or near the proximal end of the end effecter slide 16. The radiopaque markers 24, 18, 28 permit x-ray fluorescence monitoring of the precise position of the end effecter slide 16 within the vessel being treated and relative to the distal end 20 of the first guide wire 12. As will be explained below, it may be important to monitor the position of the guide wires to determine when they have been disengaged or uncoupled from each other within the vessel. In the embodiment illustrated, the radiopaque markers 24, 18, 28 comprise a gold plated surface applied to the exterior of the end effecter slide 16. However, any number of standard radiopaque marker constructions familiar to those experienced in this art would be alternatives.

As an alternative to the inclusion or use of radiopaque markers, the second wire guide apparatus 10 may comprise an overall length that is comparable to the length of the installed first guide wire 12. More specifically, and by way of example only, the second wire guide apparatus 10 may comprise a second support wire 14 having a length that is approximately equal to the length of the installed first guide wire 12. As will be explained below, the approximately equal lengths of these guide wire components allows the user to determine when the end effecter slide 16 has moved past and become disengaged from the distal end 20 of the installed first guide wire 12 by comparing the relative positions of the proximal ends of the guide wires. In other words, the end effecter slide 16 will have disengaged from the distal end 20 of the installed first guide wire 12 when the proximal end of the second guide wire apparatus 10 and the proximal end of the installed first guide wire 12 protrude equidistantly from the body of the patient.

Figure 4 is a cross-sectional view of the end effecter slide (coupling element) 16 illustrating the guide wire channel (passageway) 32 extending through the distal portion thereof. The guide wire channel 32 is sized and configured to engage and slide along the installed first guide wire 12 (Figure 2). In the embodiment illustrated, the guide wire channel 32 has a circular cross-sectional area that will slidably accommodate a typical 0,36 mm (0.014") diameter wire guide there through. For example, the guide wire channel 32 may have 0,43 (0.017") inside diameter. The guide wire channel 32 extends between a proximal opening 34 that provides access to the proximal end of the guide wire channel 32, and a distal opening 30 that provides access to the distal end of the guide wire channel 32. In the embodiment illustrated, the guide wire channel 32 has length of approximately 10 mm, although other lengths may be utilized. The end effecter slide 16 is preferably configured to resist excessive bending or articulation along the length thereof, and in particular, at or near proximal (or intermediate) opening 34. Such a configuration is intended to prevent buckling of the second guide wire apparatus 10 as it is being pushed along the installed first guide wire 12.

The end effecter slide 16 further comprises an opening or insertion channel 38 that is configured to fixedly engage with the distal end of the second support wire 14 (Figure 2) so as to for an integral structure or assembly. Connection between the insertion channel 38 and the second support wire 14 is preferably configured to resist excessive bending or articulation so as to prevent buckling of the second guide wire apparatus 10 as it is being pushed along the installed first guide wire 12. The distal end of the second support wire 14 can be affixed or bonded to the end effecter slide 16 by any number of methods known to those skilled in the art.

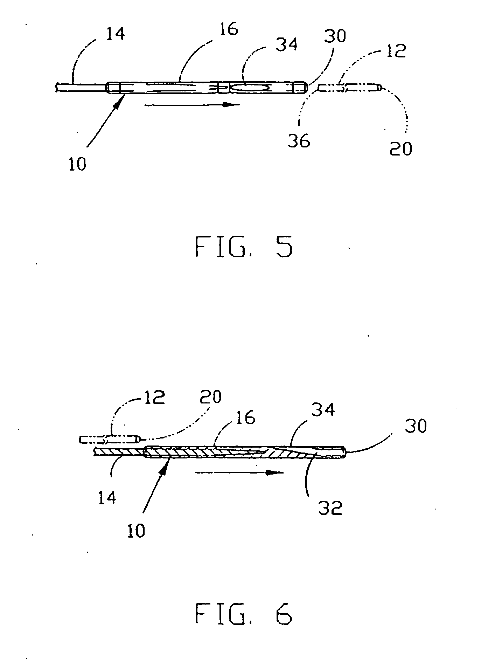

Figure 5 shows a second guide wire apparatus 10 aligned with the proximal end 36 of installed first guide wire 12 and in position to begin sliding the distal end of the end effecter slide 16 over the proximal end 36 of installed first guide wire 12. More specifically, the distal opening 30 of the end effecter slide 16 is aligned with the proximal end 36 of installed first guide wire 12 so that the proximal end 36 will pass into the guide wire channel 22 as the second guide wire apparatus 10 is moved further distally relative to the installed first guide wire 12.

Figure 6 shows end effecter slide 16 having been pushed along installed first guide wire 12 by applying a pushing force to the proximal end of second support wire 14 until it has passed beyond distal tip 20 of the installed first guide wire 12, thereby allowing the two guide wires to disengage from each other and separate. In an exemplary PTCA process, the distal ends of both of the guide wires would be past the lesion being treated.

In the embodiment illustrated in Figure 6, second support wire 14 protrudes axially approximately 15 mm (0.58 inches) into the center portion of end effecter slide 16. Second support wire 14 is typically 0.4 mm (0.014 inches) in diameter, although it could be of any diameter selected to optimize the procedure in question and with a commensurate increase in outside diameter of the end effecter slide 16. The second support wire 14 comprises a stiffness that is generally greater than that of the first installed guide wire 12. In the preferred embodiment, second support wire 14 is made from Nitinol, although in many applications other materials such as stainless steel would be sufficient. The second support wire 14 may be lubricated with silicone, PTFE or the like to facilitate its movement through the vessel. The second support wire 14 may also comprise an exterior color chosen to contrast with that of the installed first guide wire 12 to assist a user in distinguishing between the two guide wires..

The operation of the second guide wire apparatus 10 will now be described. First guide wire 12 is inserted into a vessel using standard PTCA techniques and steered with a conventional controller until distal end 20 is well past the lesion or stenosis to be treated. As shown in Figure 5, the installed first guide wire 12 is then held stationary with conventional over-the-wire techniques as the end effecter slide 16 of the second guide wire apparatus 10, with second support wire 14 attached, is slid over the proximal end 36 of installed first guide wire 12. The second support wire 14 is pushed distally relative to the installed first guide wire 12 so as to feed the end effecter slide 16 along first guide wire 12 and past the lesion or stenosis to be treated. As shown in Figure 6, the second support wire 14 is pushed further distally relative to the installed first guide wire 12 until the end effecter slide 16 is pushed off the distal end 20 of the first guide wire 12 to thereby disengage and separate the two guide wires from each other. Separation of the two guide wires can be monitored by x-ray fluorescence if the end effecter slide 16 is equipped with radiopaque markers, or may be determined by comparing the relative position of the proximal ends of the guide wires if they are of comparable length. At this point, with the two guide wires being parallel to each other with the vessel, first guide wire 12 can then be advanced further along the vessel to the next lesion or stenosis to be treated and the process repeated, or the first guide wire 12 can be retracted to allow the more substantial second support wire 14 to remain. The second support wire 14 can then become the guide wire for angioplasty balloons, stents, stent delivery devices, rotary atherectomy devices, medical catheter devices, or other bulky, less flexible, and less trackable devices.

It is therefore intended that the foregoing detailed description be regarded as illustrative rather than limiting, and that it be understood that it is the following claims that are intended to define the scope of this invention.

## Claims

1. A guide for guiding a medical device into a mammalian body, comprising:
a wire guide (14) comprising a proximal portion and a distal portion; and
a coupling element (16) fixedly integrated with said distal portion of said wire guide, said coupling clement comprising a distal end, a proximal end, and a passageway (32), **characterized in** said passageway extending between a distal opening in the distal end of the coupling element and an intermediate opening located between the distal end and the proximal end of said coupling element, said passageway being configured to slidably engage a second wire guide (12).

2. The guide of claim 1, wherein said coupling element comprises an insertion channel that is fixedly attached to a distal end of said wire guide.

3. The guide of claim 1, wherein said intermediate opening is disposed in a side of said coupling element.

4. The guide of claim 1, wherein said coupling element comprises a radiopaque marker.

5. The guide of claim 4, wherein said radiopaque marker is disposed near the distal end of said coupling member.

6. The guide of claim 5, wherein said coupling member further comprises a second radiopaque marker disposed near the intermediate opening of said coupling member.

7. The guide of claim 1, wherein said coupling member comprises a diameter that is greater than a diameter of said wire guide.

8. The guide of claim 7, wherein said coupling member comprises a chamfer at one of the proximal end and the distal end of said coupling member.

9. The guide of claim 1, wherein said coupling member comprises a stiffness that is greater than a stiffness of said wire guide.

10. The guide of claim 1, wherein said coupling member is configured to substantially not articulate along the length thereof.

11. The guide of claim 1, wherein the coupling element is configured to substantially not articulate relative to said wire guide.

12. A system for guiding a medical device into a mammalian body, comprising a guide according to any one of the preceding claims, and
a second wire guide, wherein said wire guide is configured to guide said medical device in said mammalian body.

13. The system of claim 12, wherein said second wire guide is slidably disposed through the passageway of the coupling element of said wire guide.

14. The system of claim 13, wherein the coupling element of said wire guide is configured to substantially prevent articulation of the wire guide relative to the second wire guide.

15. The system of claim 12, wherein the wire guide comprises a length that is greater than a length of the second wire guide.

16. The system of claim 12, wherein said wire guide comprises a stiffness that is greater than a stiffness of said second wire guide.

17. The system of claim 12, wherein said wire guide comprises a diameter that is greater than a diameter of said second wire guide.

18. The system of claim 12, wherein a medical device is slidably disposed about said second wire guide.

19. The system of claim 18, wherein said medical device comprises one of a medical catheter, a balloon angioplasty device, an atherectomy device, a stent delivery device, and a laser device.

## Patentansprüche

1. Führung zum Führen einer medizinischen Vorrichtung in den Körper eines Säugetieres, die Folgendes umfasst: einen Führungsdraht (14) mit einem proximalen Abschnitt und einem distalen Abschnitt; und ein Verbindungselement (16), das fest in den distalen Abschnitt des Führungsdrahts integriert ist, wobei das Verbindungselement ein distales Ende, ein proximales Ende und einen Durchgang (31) umfasst, **dadurch gekennzeichnet, dass** sich der Durchgang zwischen einer distalen Öffnung im distalen Ende des Verbindungselements und einer zwischen dem distalen Ende und dem proximalen Ende des Verbindungselements angeordneten Zwischenöffnung erstreckt, wobei der Durchgang so konfiguriert ist, dass er gleitend in einen zweiten Führungsdraht (12) eingreift.

2. Führung nach Anspruch 1, worin das Verbindungselement einen Einführungskanal umfasst, der fest an einem distalen Ende des Führungsdrahts befestigt ist.

3. Führung nach Anspruch 1, worin die Zwischenöffnung in einer Seite des Verbindungselements angeordnet ist.

4. Führung nach Anspruch 1, worin das Verbindungselement eine röntgenopake Markierung umfasst.

5. Führung nach Anspruch 4, worin die röntgenopake Markierung in der Nähe des distalen Endes des Verbindungselements angeordnet ist.

6. Führung nach Anspruch 5, worin das Verbindungselement ferner eine zweite röntgenopake Markierung umfasst, die in der Nähe der Zwischenöffnung des Verbindungselements angeordnet ist.

7. Führung nach Anspruch 1, worin das Verbindungselement einen Durchmesser umfasst, der größer ist als ein Durchmesser des Führungsdrahts.

8. Führung nach Anspruch 7, worin das Verbindungselement eine Schräge am proximalen Ende oder am distalen Ende des Verbindungselements umfasst.

9. Führung nach Anspruch 1, worin das Verbindungselement eine Steifheit umfasst, die größer ist als eine Steifheit des Führungsdrahts.

10. Führung nach Anspruch 1, worin das Verbindungselement so konfiguriert ist, dass es über seine Länge im Wesentlichen nicht gelenkig ist.

11. Führung nach Anspruch 1, worin das Verbindungselement so konfiguriert ist, dass es relativ zum Führungsdraht im Wesentlichen nicht gelenkig ist.

12. System zur Führung einer medizinischen Vorrichtung in den Körper eines Säugetieres, das eine Führung nach einem der vorhergehenden Ansprüche sowie einen zweiten Führungsdraht umfasst, worin der Führungsdraht so konfiguriert ist, dass er die medizinische Vorrichtung in dem Körper eines Säugetieres führt.

13. System nach Anspruch 12, worin der zweite Führungsdraht gleitend durch den Durchgang des Verbindungselements des Führungsdrahts angeordnet ist.

14. System nach Anspruch 13, worin das Verbindungselement des Führungsdrahts so konfiguriert ist, dass es eine gelenkige Bewegung des Führungsdrahts relativ zum zweiten Führungsdraht im Wesentlichen verhindert.

15. System nach Anspruch 12, worin der Führungsdraht eine Länge umfasst, die größer ist als eine Länge des zweiten Führungsdrahts.

16. System nach Anspruch 12, worin der Führungsdraht eine Steifheit umfasst, die größer ist als eine Steifheit des zweiten Führungsdrahts.

17. System nach Anspruch 12, worin der Führungsdraht einen Durchmesser umfasst, der größer ist als ein Durchmesser des zweiten Führungsdrahts.

18. System nach Anspruch 12, worin eine medizinische Vorrichtung gleitend um den zweiten Führungsdraht angeordnet ist.

19. System nach Anspruch 18, worin die medizinische Vorrichtung einen medizinischen Katheter, eine Ballon-Angioplastievorrichtung, eine Atherektomie-Vorrichtung, eine Stentimplantationsvorrichtung oder eine Laservorrichtung umfasst.

## Revendications

1. Guide permettant de guider un dispositif médical dans le corps d'un mammifère, comprenant :
un guide (14) de câble comprenant une partie proximale et une partie distale et
un élément de liaison (16) intégré de façon fixe à ladite partie distale dudit guide de câble, ledit élément de liaison comprenant une extrémité distale, une extrémité proximale et un passage (32),
**caractérisé en ce que**
ledit passage s'étend entre une ouverture distale prévue dans l'extrémité distale de l'élément de liaison et une ouverture intermédiaire située entre l'extrémité distale et l'extrémité proximale dudit élément de liaison et
**en ce que** ledit passage est configuré pour qu'un deuxième guide (12) de câble puisse s'y engager en coulissant.

2. Guide selon la revendication 1, dans lequel ledit élément de liaison comprend un canal d'insertion qui est attaché de façon fixe à une extrémité distale dudit guide de câble.

3. Guide selon la revendication 1, dans lequel ladite ouverture intermédiaire est ménagée dans un côté dudit élément de liaison.

4. Guide selon la revendication 1, dans lequel ledit élément de liaison comprend un marqueur opaque au rayonnement.

5. Guide selon la revendication 4, dans lequel ledit marqueur opaque au rayonnement est placé à proximité de l'extrémité distale dudit élément de liaison.

6. Guide selon la revendication 5, dans lequel ledit élément de liaison comprend de plus un deuxième marqueur opaque au rayonnement placé à côté de l'ouverture intermédiaire dudit élément de liaison.

7. Guide selon la revendication 1, dans lequel le diamètre dudit élément de liaison est plus grand que le diamètre dudit guide de câble.

8. Guide selon la revendication 7, dans lequel ledit élément de liaison comprend un chanfrein ménagé sur l'extrémité proximale ou sur l'extrémité distale dudit élément de liaison.

9. Guide selon la revendication 1, dans lequel la rigidité dudit élément de liaison est plus haute que la rigidité dudit guide de câble.

10. Guide selon la revendication 1, dans lequel ledit élément de liaison est configuré pour essentiellement ne pas s'articuler dans le sens de sa longueur.

11. Guide selon la revendication 1, dans lequel l'élément de liaison est configuré pour essentiellement ne pas s'articuler par rapport audit guide de câble.

12. Système de guidage d'un dispositif médical dans le corps d'un mammifère, comprenant un guide selon l'une quelconque des revendications précédentes et un deuxième guide de câble, ledit guide de câble étant configuré pour guider ledit dispositif médical dans ledit corps de mammifère.

13. Système selon la revendication 12, dans lequel ledit deuxième guide de câble est placé de façon coulissante dans le passage de l'élément de liaison dudit guide de câble.

14. Système selon la revendication 13, dans lequel l'élément de liaison dudit guide de câble est configuré pour essentiellement empêcher que ledit guide de câble s'articule par rapport au deuxième guide de câble.

15. Système selon la revendication 12, dans lequel la longueur du guide de câble est plus grande que celle du deuxième guide de câble.

16. Système selon la revendication 12, dans lequel la rigidité dudit guide de câble est plus haute que celle dudit deuxième guide de câble.

17. Système selon la revendication 12, dans lequel le diamètre dudit guide de câble est plus grand que le diamètre dudit deuxième guide de câble.

18. Système selon la revendication 12, dans lequel un dispositif médical est placé de façon coulissante autour dudit deuxième guide de câble.

19. Système selon la revendication 18, dans lequel ledit dispositif médical comprend un cathéter médical, un dispositif d'angioplastie à ballon, un dispositif d'athérectomie, un dispositif de placement d'endoprothèse vasculaire ou un dispositif laser.
